# EUROPEAN PATENT APPLICATION

(11) **EP 4 374 782 A1**
(43) Date of publication of application: **29.05.2024**
(21) Application number: 23211515.4
(22) Date of filing: 22.11.2023
(51) Int. Cl.: A61B 5/053, A61B 5/00, A61N 1/32

(54) **FREQUENCIES GENERATOR APPARATUS FOR ANALYSIS AND CORRECTION OF CELLULAR IMBALANCES, IN PARTICULAR OF THE HUMAN BODY**

(30) Priority: 25.11.2022 IT 202200024336
(71) Applicant: Arpamed S.r.l., 20121 Milano (MI) (IT)
(72) Inventor: ZAMBETTI, Mattia, I-20129 MILANO (MI) (IT)
(74) Representative: Metroconsult Srl

(57) **Abstract**

Described herein is an apparatus for detecting parameters of the human body, which comprises electrodes (22, 23, 24, 25) intended to be applied to a person's body, or part thereof, in order to transmit electric signals.

The apparatus comprises a pair of independent channels (C1, C2) powered by two separate and mutually isolated power supply units, for supplying power to the electrodes (22, 23, 24, 25) with alternating electric signals with frequencies in the range of 0.1 Hz to 40 MHz.

Each channel (C1, C2) comprises a respective printed circuit board (100, 200) operating as a frequency generator with integrated oscilloscope, connected to an isolator (101, 201).

The apparatus can perform frequency analyses on the human body, or part thereof, through the application of a low-intensity electric signal, which can determine any resonance peaks in discordance with the normal electric frequency absorption of the human body.

Based on the frequency analysis, the same apparatus can also be used for applying stimulation cycles by using the separate channels (C1, C2).

## Description

From a general viewpoint, the invention relates to devices for analyzing parameters of the human body by means of electrodes.

Many medical or non-medical devices are known which are used for detecting the conditions of the human body, or part thereof, via observation of physiological parameters.

One example of such devices is the electrocardiograph, which is essentially a machine with electrodes to be applied to a person's skin, which electrodes pick up the electric signals of the heart and transmit them - by means of electric wires - to the electrocardiograph. The latter then processes such signals and reproduces them on a screen or on paper in the form of an ECG graph.

The electrocardiograph can detect minimal voltage variations in the myocardial tissue (decimals of a milliVolt) directly from the patient's skin.

Similar considerations also apply to the electroencephalogram, which is a non-invasive recording of the electric activity of the encephalon, conducted by means of external electrodes. The detected electric activity is represented as a series of waves on a screen and then printed on paper or transferred to an electronic medium.

Electrocardiographs and electroencephalographs are relatively simple machines, being essentially based on the application of electrodes that are sensitive to variations in a person's physiological parameters; they are, therefore, analogue instruments, with inherently limited performance due to such characteristics.

On the other hand, more advanced and complex apparatuses are also known for detecting different physiological parameters, which are based on modern technologies such as magnetic resonance, ultrasound, X-rays or infrared rays, the Doppler effect, and so forth. Such apparatuses permit the acquisition of information about the conditions of a patient's body, or part thereof, in the form of images and/or data (e.g. blood saturation, temperature, etc.).

This information is then used by the medical or healthcare staff for making diagnostic or therapeutic evaluations.

Such apparatuses are electronic, and normally do not require the application of electrodes, but they do require the presence of skilled personnel for supervising the magnetographic, echographic, tomographic, radiographic, etc. tests.

Therefore, such apparatuses are specialized medical or diagnostic equipment, and this limits their diffusion; this is also due to the rather high costs of machines like those used for tomography and magnetic resonance tests.

The need is therefore felt for equipment permitting the detection of physiological parameters of the human body while being relatively simple, just like analogue machines such as the electrocardiograph and the electroencephalograph, but providing information not limited to just a few organs, e.g. heart or brain.

In light of the above examination, it can be stated that a technical problem tackled by the invention is to realize, or anyway provide, an apparatus for detecting physical parameters of the human body having such structural and functional characteristics as to be able to overcome the above-mentioned limitations of the prior art taken into consideration.

The idea that solves such problem is to conduct a frequency analysis on the human body, or part thereof, through the application of a low-intensity electric signal.

It is thus possible to observe the response of the human body as the frequency varies, detecting any resonance peaks in discordance with the normal electric frequency absorption of the human body.

Even more preferably, the application of the signal and the reception of the frequency response are obtained by means of electrodes applied to areas of the body, such as hands, wrists, ankles, etc.

The frequency analysis method and the related apparatus according to the invention can combine the advantages of electrode-type apparatuses, such as the electrocardiograph and the electroencephalograph, with those of electromagnetic equipment operating with high frequencies, without however the aforesaid contraindications or limitations. Furthermore, in accordance with a preferred embodiment, the method and the related apparatus according to the invention can execute a step of interacting with the person's body, or part thereof, on the basis of the results of the frequency analysis.

The features of the invention are more specifically set out in the claims appended to this description.

Such features, the effects thereof, and the results attained by the invention will become more apparent in light of the following description of one possible example of embodiment thereof as shown in the annexed drawings, which are supplied by way of non-limiting explanatory example, wherein:
- Fig. 1 shows an apparatus according to the invention, connected to an electronic computer for processing the acquired data;

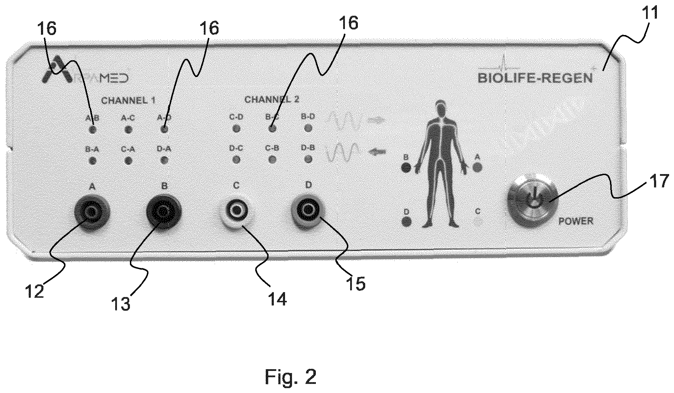

- Fig. 2 is a front view of the apparatus of the preceding figure;
- Fig. 3 is a circuit diagram of the apparatus of Fig. 1;
- Figs. 4 and 5 show graphs displayed on the screen of the electronic computer of Fig. 1, during respective operating steps of the apparatus according to the invention.

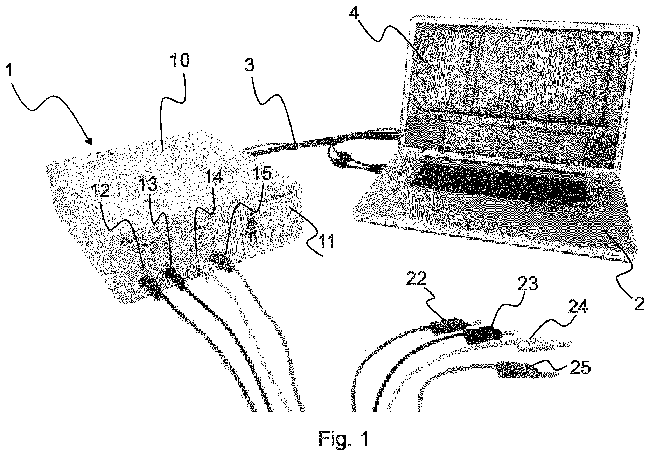

With reference to the above-listed figures, Fig. 1 illustrates an apparatus 1 according to the invention, connected to a portable electronic computer 2; the latter is a personal computer (a PC or the like) which, by means of a suitable program (i.e. software), can process the data supplied by the apparatus 1.

The program is preferably installed in the computer 2, but it may alternatively reside in a remote computer structure or platform (e.g. data center, etc.) to which the computer 2 may connect over a telecommunications network.

The computer 2 is connected to the apparatus 1 through cables 3; other implementation variants are nevertheless also possible, wherein the connection is established without using any cables 3, but using connection systems based on electromagnetic waves, such as Wi-Fi or the like.

Preferably, the computer 2 is also equipped with a screen 4 suitable for displaying graphs, diagrams, tables and other information that will be described hereinafter.

The screen 4 may be either the one of the computer 2, as in the case shown herein, or a separate screen, which may even be located in a remote location from the computer 2 and connected via a telecommunications network.

The apparatus 1 comprises a parallelepiped or box-shaped casing or external structure 10, which has a front face or wall 11 including the connection means 12, 13, 14, 15 for connecting it to external elements 22, 23, 24, 25, which will be described hereinafter. Preferably, the front face 11 houses indicator lights 16, which indicate the operating conditions, and/or one or more buttons 17, including the one for turning on the machine. The electronic components necessary for the operation of the apparatus 1 are housed inside the casing 10, which components are briefly described below.

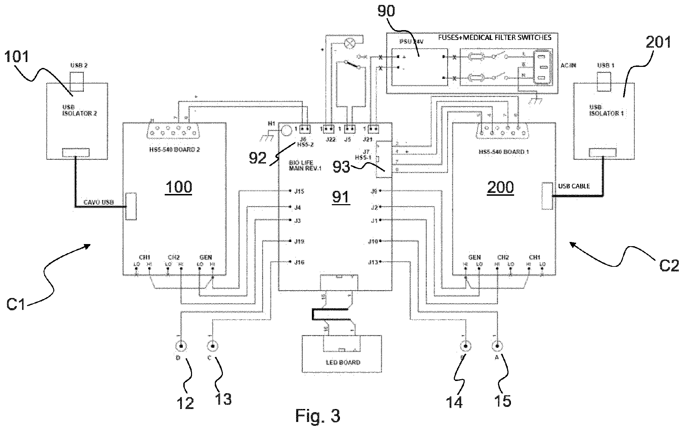

To this end, for simplicity and clarity, reference will be made to the diagram shown in Figure 3.

This should not, however, be understood as limiting or binding, nor any conclusions should be drawn by extrapolating parts of the description from the context and teaching of the present disclosure, in that the information provided herein will be sufficient for a person skilled in the art to understand the invention.

Moreover, those skilled in the art will also be able to conceive variants of the present invention as described herein.

In this frame, it must be pointed out that any reference to "an embodiment" in the present description will indicate that a particular solution, structure or feature is comprised in at least one possible embodiment of the invention.

Therefore, expressions like "in an embodiment" and the like, which may be found in different parts of this description, will not necessarily refer to the same embodiment. Moreover, any particular configuration, structure of feature may be combined as deemed appropriate in one or more embodiments, even if not expressly shown or described herein, in accordance with the teaching of the invention or the knowledge of those skilled in the art.

The reference numerals below are used only for simplicity's sake, and shall not limit the protection scope or extension of the invention and of the various embodiments thereof. With this in mind, it can be stated that, from a general viewpoint, the apparatus 1 comprises two channels C1 e C2 totally independent of each other.

Each channel C1, C2 comprises a respective printed circuit board 100, 200, preferably an HS5-540XM model manufactured by TiePie Engineering, operating as a frequency generator with integrated oscilloscope, connected to an isolator 101, 201. The latter is preferably of the USB type, e.g. the model manufactured by AMEL.

Said USB isolator 101, 201 performs the function of ensuring total isolation of the corresponding printed circuit board 100, 200 from any external power source, including the ground of the USB port, for the purpose of keeping the two above-mentioned channels C1, C2 totally independent of each other.

The printed circuit boards 100, 200 are driven by a specially designed circuit, which receives 24V power from a power supply unit (PSU), in turn receiving 220V power from the electric grid.

The unit 90 in the apparatus 1 is appropriately shielded by a steel shield (not shown in the drawings) to avoid the emission of electromagnetic noise towards the adjacent printed circuit boards 100, 200, and is equipped with a 2xMOPP double patient protection system. The electric circuit of the apparatus 1 includes a 24V power supply for a control board 91.

Said board 91 includes two DC-DC converters 92, 93, one per channel C1, C2, which generate a -12V power supply.

The control board 91 also supplies 12V power to the printed circuit boards 100 and to a series of secondary circuits that control the LEDs and relays used for switching all the necessary circuits.

For this purpose, the apparatus 1 comprises a series of sockets or ports 12, 13, 14, 15, into which corresponding jacks of electrode cables 22, 23, 24, 25 are intended to be plugged. In a preferred embodiment, the cables 22-25 are single-pole, unshielded cables, while the electrodes 22-25 are made of conductive silicone with patch gel, or hypoallergenic carbon fiber or stainless steel.

The apparatus thus equipped can emit a current with a maximum voltage of ± 12 V, with a frequency in the range of 0.1 Hz and 40 MHz, and is provided with a 270Ohm resistor for measuring the current during the application to the human body, with an output current limit of +/- 25 mA.

The whole system is controlled by specialized software, which is preferably installed in the electronic computer 2 connected to the apparatus 1.

From a functional viewpoint, it can be stated that the apparatus 1 is a system for generating electric signals with programmable frequency, equipped with a data reacquisition circuit, controlled by specific software installed in the computer 2.

It permits conducting a frequency analysis on the human body and determining any resonance peaks in discordance with the normal electric frequency absorption of the human body, by reading a pair of electrodes 22, 23 or 24, 25 normally applied to the patient's hands, wrists or ankles.

According to a preferred embodiment, the operation of the apparatus 1 includes a subsequent operating step, which is conducted with the electrodes 22-25 applied to the ends of the four limbs of the patient and using both channels C1 and C2, which remain totally independent even when used simultaneously, by transmitting a weak current with specific frequency and intensity, calculated on the basis of the frequency response. To this end, the values of the electric signal are processed by the program installed in the computer 2.

The Applicant observed that this subsequent step makes it possible to normalize any imbalances detected in the previous frequency analysis step conducted on the body, or part thereof, thus promoting a general re-balancing of the body and improving the patient's condition by means of a non-invasive treatment.

Normally, during the first step of subjecting a person's body, or part thereof, to a frequency analysis, at least two electrodes 22-25 are connected to the patient's body and a range of frequencies of interest is investigated which may vary, at most, between 0.1 Hz and 40 MHz, with suitable step/time, in order to scan the desired frequency interval. During this operating step, only one channel C1 or C2 of the apparatus is active, which allows drawing Hz-mA and Hz-Ohm diagrams, with a bio-feedback circuit.

Preferably, a suitable mathematical processing includes data analysis and fitting procedures to highlight any areas of "abnormal" absorption in terms of frequency, characterized by resonance peaks at specific frequencies.

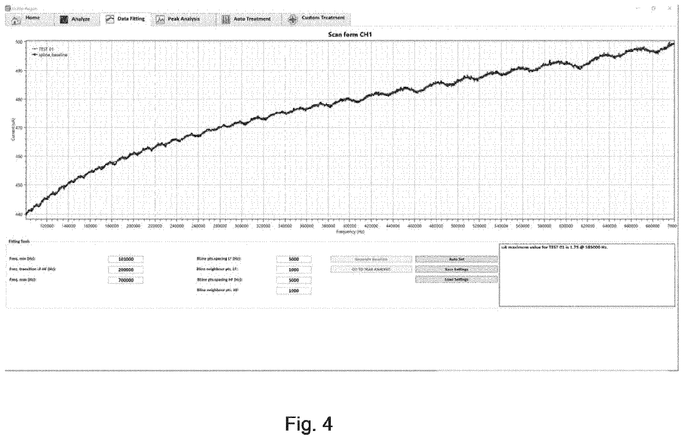

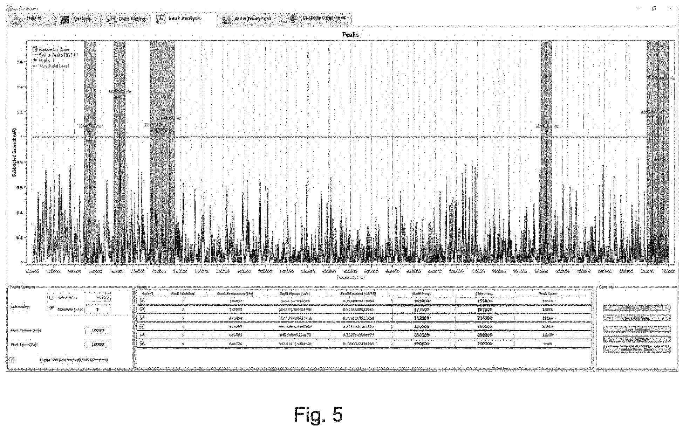

Figures 4 and 5 show respective graphs that can be displayed on the screen 4 of the computer 2, wherein the first one concerns a data fitting verification, while the second one illustrates the analysis of the peaks originated from the frequency response of a person's body.

As can be appreciated, the information provided by the frequency analysis is indicative of the person's general physical and/or biological parameters, obtained through the use of electrodes.

Such information is not, therefore, limited to a specific organ, like the heart and the brain for, respectively, the electrocardiograph and the electroencephalograph, since it consists of readings that may concern a whole range of different parameters, such as electrodynamic and energetic parameters, of a person's body.

This information can then be evaluated or studied by skilled healthcare personnel, scientists, or the like, for diagnostic or other purposes.

For example, the Applicant unexpectedly observed that, based on the frequency response data obtained in the first step, it was then possible to promote cellular energetic re-balancing to re-balance the whole body and improve the patient's condition by means of a non-invasive treatment.

Generally, during this step both channels C1 and C2 are active in the apparatus 1, and four electrodes 22-25 are connected to the person's body, preferably to end regions of the limbs; normally a pair of electrodes 22, 23 are applied to the hands, while the other pair 24, 25 are applied to the feet, and through them the signals acquired during the previous frequency analysis step are sent, with appropriate waveforms, amplitude and phase, in order to obtain a re-balancing effect on the human body.

With an additional subsequent frequency analysis like the previous one already described above, one may verify the efficacy of the administered treatment; further cycles of such alternated analysis and treatment steps may then be conducted.

From the above description one can understand how the apparatus 1 according to the invention solves the technical problem outlined at the beginning.

This is a relatively simple apparatus, from both the structural and functional viewpoints. As to the former aspect, the apparatus 1 is similar to electrode-based machines like electrocardiographs, as previously described herein. Therefore, it is relatively simple and can be manufactured at low cost using commercially available components.

From the functional viewpoint, the apparatus 1 can offer high performance, like the analysis of the frequency response of a person's body, or part thereof, to which voltage and/or current signals are applied (through the electrodes 22-25) which have a low intensity but a wide range of frequencies, variable between 0.1 Hz and 40 MHz.

This derives from the fact that, as previously explained, in the apparatus 1 the two channels C1 and C2 are totally isolated from each other electromagnetically, thus preventing any interference from occurring and thereby ensuring high precision and proper control of the transmission and reception of the electric signals applied by means of electrodes.

From a functional viewpoint, therefore, useful information can be obtained about a person's electro-physiological characteristics, which can then be used for several applications, e.g. a treatment that may have a positive effect at cellular level, resulting in an improved condition of the patient's body.

It is just worth adding that the use of the electrodes 22-25 facilitates the application of low-intensity electric signals, resulting in no contraindications for the person involved. This is not always true for some prior-art diagnostic or medical machines, such as radiographic or tomographic ones.

As aforesaid, those skilled in the art will be able to make modifications to the above-described circuit configuration of the machine 1.

For example, the number of electrodes may be different (either greater or smaller) than the one considered herein, and so may the number of channels C1, C2. Also the various electronic components may be modified as necessary.

All such features shall nevertheless fall within the scope of the following claims.

## Claims

1. Apparatus for detecting parameters of the human body, comprising a plurality of electrodes (22, 23, 24, 25) intended to be applied to a person's body, or part thereof, in order to transmit electric signals, means (C1, C2, 90, 91, 92, 93, 100, 101, 200, 201) for supplying power to the electrodes (22, 23, 24, 25), **characterized in that** the electric signals are of the alternating type.

2. Apparatus according to claim 1, wherein the frequencies are in the range of 0.1 Hz to 40 MHz.

3. Apparatus according to claims 1 or 2, wherein said power supply means (C1, C2, 90, 91, 92, 93, 100, 101, 200, 201) comprise at least one pair of independent channels (C1, C2).

4. Apparatus according to claim 3, wherein each channel (C1, C2) comprises a respective printed circuit board (100, 200) operating as a frequency generator with integrated oscilloscope, connected to an isolator (101, 201).

5. Apparatus according to claim 4, wherein the isolator (101, 201) is of the USB type.

6. Apparatus according to claims 4 or 5, wherein the isolator (101, 201) performs the function of ensuring total isolation of the corresponding printed circuit board (100, 200) from any external power source, for the purpose of keeping the two above-mentioned channels (C1, C2) totally independent of each other.

7. Apparatus according to any one of claims 4, 5, 6, wherein the printed circuit boards (100, 200) are driven by a circuit that receives power from a power supply unit (90), in turn receiving power from the electric grid.

8. Apparatus according to claim 7, wherein the power supply unit (90) is shielded by a metal plate or the like, in order to avoid the emission of electromagnetic noise towards the adjacent printed circuit boards (100, 200).

9. Apparatus according to any one of claims 3 to 8, comprising a printed circuit board (91) equipped with two direct-current converters (92, 93), one per channel (C1, C2), for acquiring the reading of the feedback current received from the human body.

10. Apparatus according to any one of claims 3 to 9, wherein the channels (C1, C2) are adapted to emit and/or receive electric signals, respectively.

11. Method of frequency analysis of the human body, or part thereof, comprising a step of applying low-intensity electric signals by means of electrodes (22, 23, 24, 25), with frequencies in the range of 0.1 Hz to 40 MHz.

12. Method according to claim 11, comprising an operating step of subsequently applying an electric signal having specific frequency and intensity determined on the basis of the frequency response.

13. Method according to claims 11 or 12, wherein the steps of analyzing the frequency response and subsequently applying the electric signal are performed by using respective and mutually separate channels (C1, C2) for supplying power to the electrodes (22-25).
